# EUROPEAN PATENT SPECIFICATION

(11) **EP 3 571 510 B1**
(45) Date of publication and mention of the grant of the patent: **19.05.2021**
(21) Application number: 18700439.5
(22) Date of filing: 22.01.2018
(51) Int. Cl.: G01N 33/80, B01L 3/00

(54) **CENTRIFUGAL IMMUNOASSAY METHODS AND DEVICES**
ZENTRIFUGISCHER IMMUNOASSAY-VERFAHREN UND -VORRICHTUNGEN
PROCÉDÉS ET DISPOSITIFS DE DOSAGE IMMUNOLOGIQUE CENTRIFUGIQUE

(30) Priority: 20.01.2017 EP 17152516
(43) Date of publication of application: 27.11.2019
(73) Proprietor: Université Libre de Bruxelles, 1050 Bruxelles (BE)
(72) Inventor: CORAZZA, Francis, 1030 Schaerbeek (BE); EL KENZ, Hanane, 1020 Laeken (BE); BALTUS, Thierry, 4537 Verlaine (BE)
(74) Representative: Brantsandpatents bvba
(86) International application number: PCT/EP2018/051393
(87) International publication number: WO 2018/134387

(56) References cited:
- WO-A1-93/08893
- WO-A1-2011/088582
- US-A- 5 173 193
- US-A1- 2003 224 457
- US-A1- 2004 265 172
- US-A1- 2014 296 050

## Description

### TECHNICAL FIELD

The invention pertains to devices for performing immunoassay tests, in particular devices for immunoassay tests based on agglutination. In addition, the invention provides methods for conducting said immunoassay tests.

### BACKGROUND

Immunoassay tests have been widely used in many important medical areas such as diagnosis of diseases, therapeutic drug monitoring, clinical pharmacokinetic and bioequivalence studies in drug discovery and pharmaceutical industries. The analysis in these areas usually involves the detection and/or the measurement of very low concentrations of macromolecular biomolecules or small molecules, metabolites, and/or biomarkers. The importance and widespread of immunoassay tests are attributed to their inherent specificity, high-throughput, and high sensitivity for the analysis of wide range of analytes in biological samples such as in blood.

Blood transfusions are used for various medical conditions to treat blood loss or to supply certain blood components. Before a recipient receives a transfusion, cross matching between donor and recipient blood must be done. In certain situations, for example the treatment of an emergency patient, obtaining a rapid cross matching result or rapid identification of the blood group is very important for rapid treatment of the patient. Thus, there is a need to develop methods and devices that can rapidly perform and provide blood cross matching results.

ABO incompatibility still represents one of the most threatening risks in transfusion medicine. It is most often the result of human error associated with a failure in organization. Bedside testing performed immediately prior to transfusion might prevent this complication and is therefore required by law in many countries. Despite the availability of the bedside tests, erroneous transfusions still occur. The available bedside tests are generally test cards on which manual testing of blood groups is performed by a user, generally a nurse. After depositing the blood samples to be tested, the user visually interprets the results to ascertain the compatibility of the tested blood samples. The critical issue in current manual bedside testing for ABO blood compatibility is the accuracy of the test and the human interpretation of the results, depending markedly on correct use of the test cards.

In 2008, the Food and Drug Administration (FDA) reported that 7% of transfusion-related deaths were attributable to ABO-associated haemolytic reactions. Linden et al. [Transfusion 2000; 40:1207-1213] observed that one error-related ABO-incompatible transfusion occurred every 38 000 RBC units transfused in New York State. The Haemovigilance network in France showed similar findings from 1994 to 1998 and estimated the risk of death due to ABO-mismatched transfusion at 1:1 800 000 allogeneic red cell units transfused [Andreu et al. Transfusion 2002; 42:1356-1364]. US 2003/224457 relates to an apparatus, for determining a blood group type of an individual, comprising an optical bio-disc including at least one capture chamber including: a layer including a first capture antibody, and a layer including a second capture antibody bound by the first capture antibody, the second capture antibody is specific for a blood group antigen, a disc drive assembly; an optical reader; and software for blood group analysis.

Since 1985, the French authorities have introduced a systematic bedside ABO agglutination test for checking that the right blood is given to the right patient. Consequently, the incidence of ABO-incompatible transfusions has decreased to 1:235 000 [Durat, Transfus Clin Biol 2008; 15:322-326]. Their experience underlines the efficiency of bedside verification of ABO group on both patient and donated blood. However, this strategy requires an extremely time-consuming learning program and still relies on a human interpretation of bedside card ABO agglutination test.

It is the aim of the current invention to provide an automatic bedside testing device and method which overcome at least part of the above mentioned drawbacks and disadvantages. One object of the invention is to provide an automatic bedside testing device and method which is devoid of human results interpretation and which allow rapid and reliable immunoassay tests such as blood group identification tests.

### SUMMARY OF THE INVENTION

In a first aspect, the present invention provides a device for performing immunoassay tests comprising:
- a readable device for performing immunoassay tests comprising a platform which comprises at least two sample receiving means for receiving at least one liquid biological sample from at least one donor and at least one donor differentiation means for differentiating different donor types; each sample receiving means is fluidly connectable to at least two test units, each of said test units comprises at least one reagent incubation chamber which is fluidly connected to at least one separation means, said separation means is fluidly connected to at least one detection chamber, characterized in that the platform comprises at least one volume indication means for detecting the presence of a predetermined volume of the liquid biological sample.
- a reading device for performing immunoassay tests comprising at least one housing in which an aforementioned readable device is insertable, said housing is connected to at least one rotational means for rotating the readable device at a predetermined rotation speed; a lid movable between an open position in which the readable device is visible and a closed position in which the readable device is not visible; at least one recognition means for recognizing the presence of the readable device and/or for extracting information from the legible identification means of the readable device; at least one first optical sensor which detects the presence of at least one binding pair of molecules in the separation means of the readable device and/or the presence of free molecules in the detection chamber of the readable device, and; at least one second optical sensor which detects the presence of a liquid biological sample in the overflow chamber of the readable device.

In a second aspect, the present invention provides a method for performing immunoassay tests comprising the steps of:
a) introducing at least one liquid biological sample in the sample receiving means of a readable device according to any embodiment of the invention,
b) inserting the readable device in the housing of a reading device according to any embodiment of the invention, steps (a) and (b) being interchangeable,
c) flowing the liquid biological sample in the test units by rotating the readable device by the rotation means of the reading device, and
d) determining the result of the immunoassay test by the first optical sensor of the reading device, said first optical sensor detects presence of at least one binding pair of molecules in the separation means of the readable device and/or the presence of free molecules in the detection chamber of the readable device,
wherein the volume of the liquid biological sample introduced in the sample receiving means is detected by:
- the visual volume indication means of the readable device, and/or
- the electronically legible volume indication means of the readable device and the second optical sensor of the reading device.

The reading device, readable device and the method of the invention present several advantages compared to the devices and the methods of the prior art. The volume indication means of the readable device provides for the control of the volume of liquid biological sample prior to insertion of the readable device in the reading device. Additionally, the second control of the volume introduced in the readable device by the reading device ensures that (i) the test will be performed when a sufficient volume of liquid biological sample is present in the readable device and/or (ii) the test is run a second time if insufficient volume of liquid biological sample is present in the readable device. This double control of the sample volume ensures that accurate tests will be performed thereby considerably limiting and even preventing mistakes leading to wrong results.

In addition, the reading device guides the user by instructing him with respect to the different steps that should be performed. In a particular, the reading device informs the user that an insufficient volume of sample has been introduced in the reading device prior and/or after running the test. This allows the user to introduce additional volume of sample in the reading device or perform a second test to ensure obtaining reliable results. The additional volume of liquid biological sample can be introduced by the user through the closable opening of the lid of the reading device. The readable device is then maintained in the reading device which reduced the risk of dropping the readable device while unloading and loading it in the reading device. Furthermore, the reading device aligns the sample receiving means in which the additional sample volume needs to be introduced with the closable opening of the lid. This avoids addition of the sample in the wrong sample receiving means thereby mixing the different tested samples.

Other advantages of the reading device, the readable device and the method of the invention will be further detailed in the description below and the accompanying figures.

### DESCRIPTION OF FIGURES

**Figure 1** shows an embodiment of the readable device wherein the sample receiving means have different protruding shapes.
**Figure 2** shows an embodiment of the readable device wherein the sample receiving means have similar protruding shapes.
**Figure 3** shows an embodiment of the readable device wherein the sample receiving means have similar protruding shapes.
**Figure 4** shows an embodiment of the readable device wherein the sample receiving means have similar non-protruding shapes.
**Figure 5** shows an embodiment of the readable device wherein the sample receiving means have different protruding shapes and visual donor differentiation means.
**Figure 6** shows an embodiment of the readable device in which the receiving chambers are filled with blood.
**Figure 7** shows an embodiment of the readable device in which the distribution chambers of the test units are filled with blood.
**Figure 8** shows an embodiment of the reading device in which a readable device is inserted.

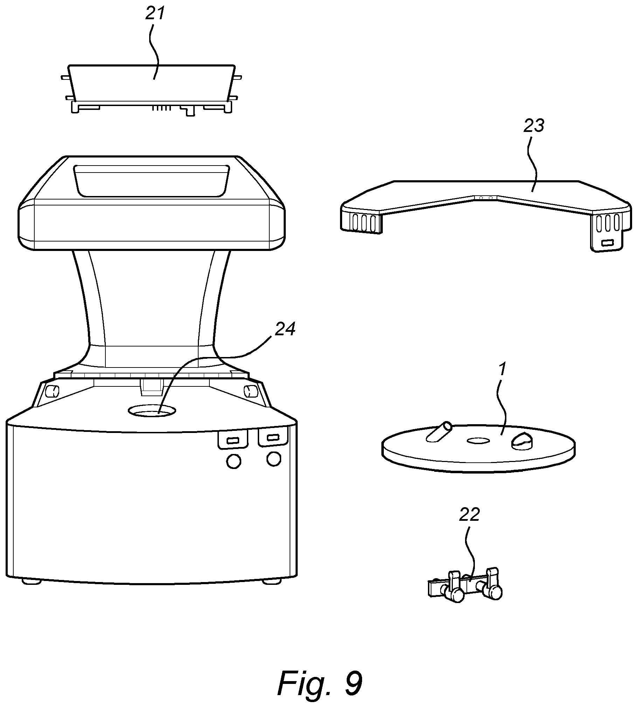

**Figure 9** shows an exploded view of the reading device of Figure 8.

### DETAILED DESCRIPTION OF THE INVENTION

The present invention concerns bedside testing, also called point-of-care testing (POCT), devices and a method which allow rapid and reliable immunoassay tests such as blood group identification tests. The device of the invention allows, for instance, the identification of the blood groups to which at least one tested blood sample belongs. The device further allows conducting blood cross matching tests prior to blood transfusion for instance.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise," "comprising," and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.
The recitation of numerical ranges by endpoints includes all numbers and fractions subsumed within that range, as well as the recited endpoints.

The present invention is related to a method for assaying (detecting the presence) an analyte present (artificially or naturally) in a liquid biological sample, said analyte being a first member of a binding pair of molecules, said binding pair of molecules comprising an antigen (Ag) and an antibody (Ab) binding to said antigen. The antigen might be present on erythrocytes of a blood sample.

Liquid biological sample refers to any liquid sample comprising an antigen and derived from a plant species, an animal or a human being. The liquid biological sample might be unprocessed or processed. By processed reference is made to a solid sample that is transformed into a liquid sample by any means known to the person skilled in the art. Said liquid biological sample include and is not limited to sweat, urine, whole blood and plasma.

Aspects of the invention preferably relate to assaying a blood group Ag or an Ab binding to a blood group Ag. Aspects of the invention preferably relate to assaying blood group antigens or antibodies binding to blood group antigens. The latter might be natural or artificial and are in fact at the surface of the erythrocytes, id est RBCs, membrane antigens, in particular blood group (or system) antigens or viral antigens, capable of being recognized by the immune system. A blood group antigen present on erythrocytes is intended to mean any antigen of the non- exhaustive list of the ABO system with the A antigen, the B antigen, the A and B antigens expressed simultaneously or the H antigen, of the Rhesus system with the D, E, e, and C or c antigens, of the Kell system with the K or k antigen, of the Duffy system (Fya, Fyb), of the Kidd system (Jka, Jkb) system or else of other systems, such as MNSs, Lewis, Lu, P1 , Lea, Leb, Cw, M, N, S, s etc.

The most clinically significant Ag system is the ABO RBC Ag system, which is unique inasmuch as the majority of human individuals produce Abs to those Ags without having been actively immunized against them. Thus, an individual's RBCs may display either A, B or both A and B Ags. About 40% of the population does not carry either of these Ags and is therefore, typed or grouped as O or Zero. The plasma or serum in blood of group O individuals has Abs against both A and B group Ags. The plasma or serum in blood of group AB individuals, however, does not demonstrate Abs to either A or B group Ags. Accordingly, the plasma or serum in blood of group A individuals has Abs against B group Ags, while the plasma or serum in blood of group B individuals has Abs against A group Ags.

Incompatibility in the ABO Ag system will result in strong adverse reactions, which can be prevented by matching the first individual e.g. the donor to the second individual e.g. the recipient. Ideally, the donor and recipient should belong to the same blood group; however, in the absence of an identical donor, an alternative blood group may be suitable as long as the recipient serum or plasma does not carry natural Abs against the donor RBCs. Thus O group is the universal donor, since its RBCs will not react with either anti-A or anti-B Abs, which are present in the blood of all other groups. Individuals of group AB can receive blood from all blood groups, since they do not have Abs to any of them.

The RBCs of an individual can either carry the rhesus (Rh) (or D) antigen (Ag) (Rh<+> or Rh-positive) or not carry it (Rh -> or Rh-negative). Unlike the ABO Ag system, anti-Rh (anti-D) Abs are not normally present in the blood Rh-negative individuals. Such Abs nevertheless develop in Rh-negative individuals following an immunological potentiation resulting from a transfusion with Rh-positive blood or from pregnancy with an Rh-positive fetus. Matching of Rh between donor and recipient, in addition to ABO matching, can be done to prevent generation of Rh Abs in Rh-negative individuals and to prevent adverse reactions in individuals that may carry anti-Rh Abs.

Besides the A, B and Rh Ags, RBCs may carry a variety of other Ags, which are sometimes referred to as sub-groups. Similarly to the Rh Ag (and to most Ags in nature), Abs to those Ags are not normally present in human blood, but may arise due to previous blood transfusions or pregnancy with an Ag-carrying fetus. Such Abs are referred to as unexpected.

Although the invention will be further described in relation to blood samples, it is to be understood that any other liquid biological sample might be used with the devices and/or the method of the present invention.

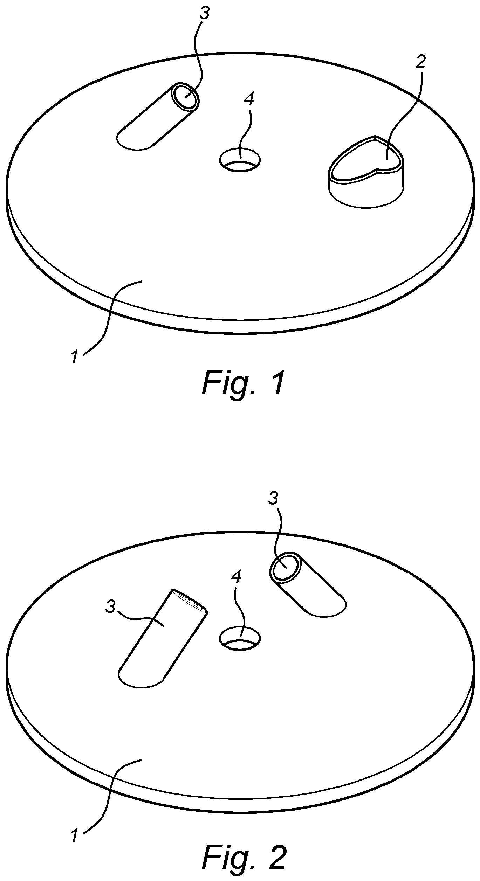

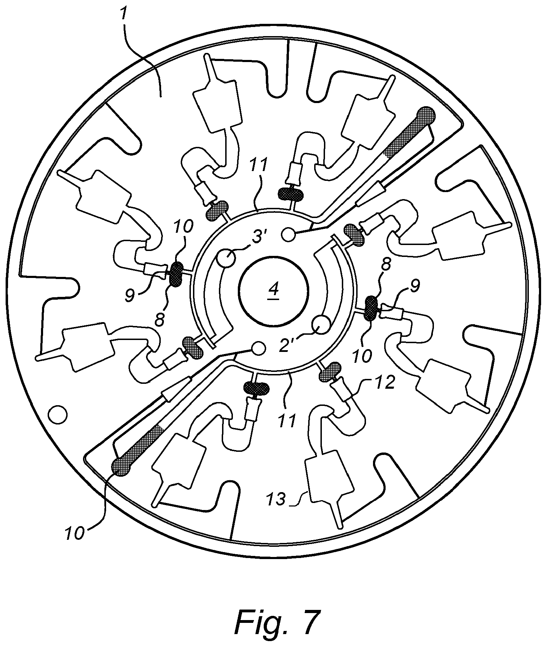

In a first aspect, the present invention provides a device for performing immunoassay tests comprising:
a readable device 1 for performing immunoassay tests comprising a platform which comprises at least two sample receiving means 2, 3, 2', 3' (figures 1 to 5) for receiving at least one liquid biological sample from at least one donor and at least one donor differentiation means for differentiating different donor types; each sample receiving means is fluidly connectable to at least two test units, each of said test units comprises at least one reagent incubation chamber 12 which is fluidly connected to at least one separation means, said separation means is fluidly connected to at least one detection chamber 13 (figure 7), characterized in that the platform comprises at least one volume indication means for detecting the presence of a predetermined volume of the liquid biological sample.
a reading device 20 for performing immunoassay tests comprising at least one housing 24 in which an aforementioned readable device is insertable, said housing 24 is connected to at least one rotational means for rotating the readable device at a predetermined rotation speed; a lid 23 movable between an open position in which the readable device is visible and a closed position in which the readable device is not visible; at least one recognition means for recognizing the presence of the readable device and/or for extracting information from the legible identification means of the readable device; at least one first optical sensor which detects the presence of at least one binding pair of molecules in the separation means of the readable device and/or the presence of free molecules in the detection chamber of the readable device, and; at least one second optical sensor which detects the presence of a liquid biological sample in the overflow chamber of the readable device.

The readable device of the invention might comprise a single platform comprising at least two sample receiving means. Each sample receiving means is permanently connected or removably connectable to the single platform and is fluidly connected to at least two test units. The test units are as described above and are comprised in the single platform.

The readable device of the invention might also comprise a first platform comprising at least two sample receiving means. Each sample receiving means is permanently connected or removably connectable to the first platform. The device further comprises a second platform comprising the test units. Said first and second platform are connectable to each other thereby fluidly connecting each sample receiving means to at least two test units. The test units are as described above. The first and second platforms are preferably mechanically connectable to each other.

In a preferred embodiment, the removably connectable sample receiving means of the readable device can be clipped onto the aforementioned first platform of the readable device in order to prevent the unwanted release of the removably connectable sample receiving means, thereby securing the fluid connection.

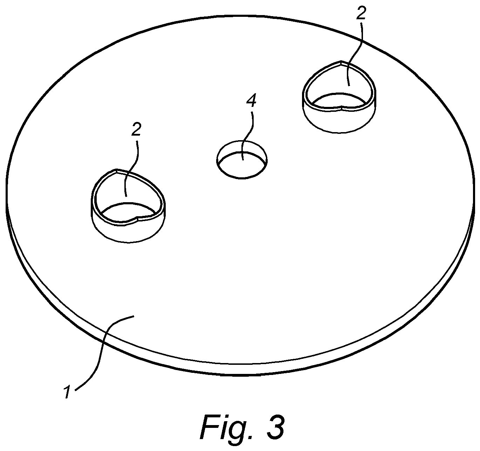

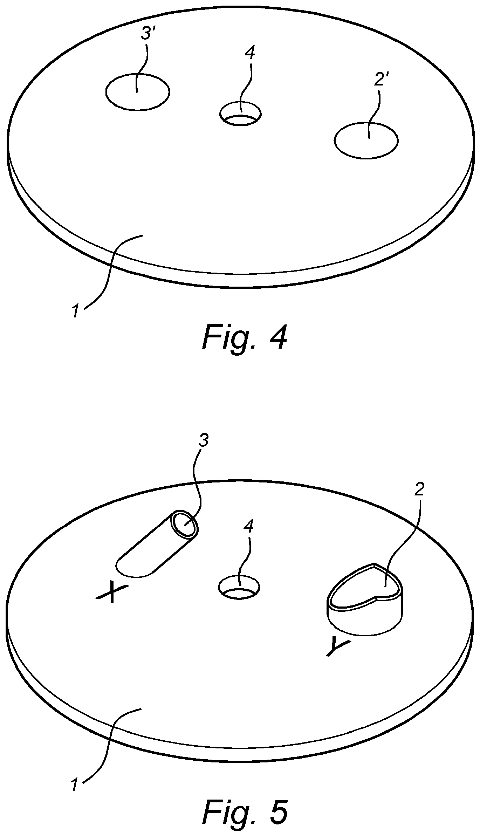

The sample receiving means of the readable device might have identical (figures 2, 3 and 4) or different shapes (figure 1). Preferably, readable devices with sample receiving means of different shape are designed for testing pre-transfusion blood group matching between two blood samples, i.e. a patient blood and a blood bag. Readable devices with sample receiving means of similar shape (figures 2 and 3) are preferably designed for testing two separate patient's blood or two blood samples from two different blood bags. The shape of each sample receiving means might be a shape that protrudes from the platform (2 and 3 in figures 1, 2 and 3) or a shape devoid of protrusion (2' and 3' in figure 4) from the platform.

Each sample receiving means of the readable device optionally comprise at least one anticoagulant agent which is in liquid or lyophilized form. Each sample receiving means might comprise at least one blood sampling means which allows sampling blood from a blood bag and/or directly from the patient (for instance by finger puncture). The blood sampling means can be a bevel, a tube segment opener, a needle or any other means known to the person skilled in the art and intended to safely withdraw blood from the donor. The blood sampling means can be embedded in or removable from the sample receiving means. The shape of the blood sampling means is selected from the group comprising an opening, a tube having any shape, a tube having any shape and comprising a blood sampling means, an open cavity having any shape in which blood can be deposited, an open cavity having any shape in which blood can be deposited by finger puncture of a patient, a tubular segment including a needle or any other system intended to safely and specifically withdraw blood from a blood bag tubing (by blood tubing piercing).

In a preferred embodiment, the platform of the readable device comprises at least one blood volume indication means which is fluidly connected to the sample receiving means. The blood volume indication means might be a visual means which is visible to the user and/or an electronically legible volume indication means which is readable by a suitable reading device. The blood volume indication means allows the user to control whether a predetermined blood volume has been introduced in the sample receiving means. This is advantageous as it avoids false results in the event insufficient blood volume for conducting accurate test is provided in the sample receiving means. The predetermined blood volume is the minimal volume of blood that should be introduced in the sample receiving means for conducting a complete blood test. Said predetermined blood volume is of from 1 µl to 250 µl, preferably from 5 µl to 200 µl, more preferably from 10 µl to 150 µl, even more preferably from 15 µl to 100 µl, most preferably from 20 µl to 50 µl, even most preferably about 25 µl.

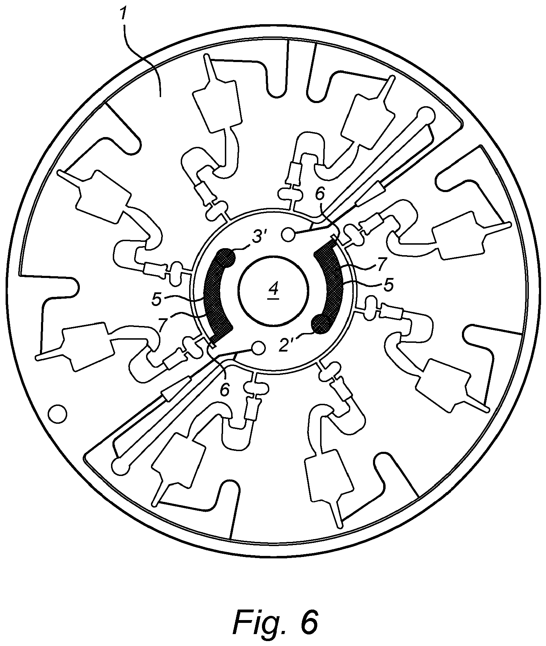

The visual blood volume indication means is preferably a receiving chamber 5 which is fluidly connected to sample receiving means 2', 3' as shown in figure 6. The receiving chamber is preferably positioned in a transparent area 14 of the readable device 1 and thereby visible to the user. The chamber comprises one inlet and one outlet. The inlet is fluidly connected to the sample receiving means for receiving the blood introduced therein. The outlet is preferably closed by a first blocking means 6. Said blocking means can be a capillary valve, a disruptable membrane or any other means known to the person skilled in the art. The blood 7 introduced by the user in the sample receiving means flows into and rests in the receiving chamber 5. The receiving chamber might be designed such that the volume of its inner space is at least equal to the predetermined blood volume required for conducting the test. A completely filled receiving chamber is thereby visible to the user and provides said user with a visual indication that the blood test can be initiated. The receiving chamber 5 might also comprise a visual mark which indicates to the user that the predetermined blood volume has been introduced and that the blood test can be initiated.

The electronically legible blood volume indication means comprises at least one overflow chamber (10 in figure 7) which is fluidly connectable or connected to the sample receiving means 2', 3' and/or the receiving chamber 5. The blood volume introduced in the sample receiving means is preferably higher than the blood volume required for accurately conducting a blood test in each test unit of the readable device. Preferably each test unit comprises a distribution chamber 8 fluidly connected to the receiving chamber 2', 3' and the overflow chamber 10. The blood sample introduced in the sample receiving means flows into the receiving chamber and is then distributed into the different distribution chambers 8 of the test units. Distribution of the blood into the distribution chambers occurs by rotating the readable device at a predetermined speed which is sufficient to flow the blood sample through the first blocking means 6 of the outlet of the receiving chamber. The blood overflow is directed to and rests in the overflow chamber 10 by at least one channel 11. Preferably, said overflow chamber is optically readable by a suitable reading device, preferably by at least one diode of said reading device. The presence of blood in the overflow chamber (as shown in figure 7) indicates that sufficient blood volume has been introduced for conducting the blood tests in all test units.

In a preferred embodiment, each distribution chamber is provided with an inlet and an outlet. The inlet is in fluid communication with a receiving chamber of the readable device. The outlet is preferably closed by a second blocking means 9 which can be similar to or different from the first blocking means 6 described above. Blood flows through the second blocking means 9 at a predetermined rotation speed which is might be similar or different from the predetermined rotation speed required for flowing blood through the first blocking means 6 which of the outlet of the receiving chambers. Blood flows through the second blocking means 9 into the reagent incubation chamber.

Preferably, the reading device provides a signal to the user after optically reading the overflow chamber. Said signal can be either a positive signal which informs the user that the test can be started or a negative signal which informs the user that the test cannot start due to insufficient blood volume in the readable device. The reading device instructs the user to introduce additional blood volume in the reading device. The additional blood volume can be introduced by the user through the closable opening of the lid of the reading device. The readable device is then maintained in the reading device which reduced the risk of dropping the readable device while unloading and loading it in the reading device. Furthermore, the reading device aligns the sample receiving means in which additional blood needs to be introduced with the closable opening of the lid. This avoids addition of blood in the wrong sample receiving means thereby mixing the different tested blood samples. Preferably, after introducing additional blood volume in the appropriate sample receiving means of the readable device, the overflow chamber is again optically read by the suitable reading device to ensure that a sufficient blood volume has been introduced.

Alternatively, the overflow chamber is optically read after the completion of the test. A negative signal is provided if no biological sample is detected in said chamber thereby providing indication to the user that the test should be performed a second time. If a part of the biological sample is detected in the overflow chamber, a positive signal is provided to the user thereby informing him that a sufficient volume of biological sample has been introduced in the readable device and the test has been successfully performed.

The combination of visual and electronically legible blood volume indication means provides the user with a double control of the blood volume introduced in the readable device prior to and/or after the test has been performed thereby avoiding waste of time and/or erroneous results.

In a preferred embodiment, the readable device comprises at least one donor differentiation means which is a visual means visible to the user and/or an electronically legible differentiation means readable by a suitable reading device. In a preferred embodiment, the electronically legible differentiation means is a barcode, a ship, a position indicator or any position identification means readable by a suitable reading device.

The visual donor differentiation means can be the shape of the sample receiving means (2 and 3 in figure 1) and/or a visual mark on the platform comprising the sample receiving means (X, Y in figure 5). The visual mark can be the shape of the sample receiving means. Preferably, the sample receiving means for testing a patient's blood sample is different from the shape of a sample receiving means of for testing blood from a blood bag. This provides visual guidance to the user and prevents confusion of blood samples to be introduced or already introduced in the readable device. The visual mark can also be selected from the group comprising a figure, a color, a letter, a text or any combination thereof. Figure 5 shows an example of a visual donor differentiation means which is a combination of sample receiving means having different shapes and different visual marks in the form of letters X and Y.

It is to be understood that donor refers to a patient or to a blood bag. The readable device allows blood group identification of blood from a patient, a person or from a blood bag. Preferably, the readable device allows ABO cross-matching between two blood samples. The latter might originate from a person and a blood bag.

The reagent incubation chamber 12 comprises at least one antibody in liquid and/or lyophilized and/or encapsulated and/or micro-encapsulated and/or coated form. Said antibody is able to bind to an antigen present on erythrocytes or any particle of the tested blood sample. At least one channel is provided between the reagent incubation chamber 12 and the separation means thereby fluidly connecting them. Similarly, at least one channel is provided between the separation means and the detection chamber 13 thereby fluidly connecting them. In a preferred embodiment, the channel connecting the reagent incubation chamber 12 to the separation means has a shape that promotes the mixing of the blood sample with the reagent. Said shape might be a serpentine shape or any other shape known to the person skilled in the art.

In a preferred embodiment, the separation means is selected from the group comprising a filtration membrane. Preferably, the filtration membrane or a layer thereof is selected from the group comprising woven tissue, non-woven tissue, and porous polymeric film such as track-etched membrane or a combination thereof. Woven tissue reduces production costs by being cheap. Woven tissue has well defined pores. Porous polymeric films and track-etched membranes have the further advantage of being less sensitive to deformation, for example induced by the assembly process. Preferably, the filtration membrane of the current invention consists of one single layer. Eventually, the filtration membrane may be mechanically supported, for example by a grid.

Advantageously, the filtration membrane of the readable device is a polymeric membrane, the polymer being preferably selected from the group consisting of nylon, cellulose-ester polymer, nitrocellulose, polyvinylidene fluoride (PVDF), polytetrafluoroethylene (PTFE), polyether sulfone, polycarbonate, polyester and a combination thereof.

The porosity of the filtration membrane of the readable device is preferably monodispersed. By having a well-defined size porosity, monodispersed membranes advantageously present a well-defined pass-through cutoff, thereby increasing the reliability of the method of the invention. Track-etched membranes present such monodispersed porosity, with the further advantage of having narrow size dispersion.

The filtration membrane of the readable device has a porosity chosen to be permeable to free erythrocytes and impermeable to hemagglutinated erythrocytes. The pores of the filtration membrane are of from 1 µm to 30 µm, preferably from 5 µm to 25 µm, more preferably from 10 µm to 20 µm, most preferably from 11 µm to 15 µm. Indicated upper and lower limits can be combined to yield optimal porosity ranges, depending on the erythrocyte size in the biological sample (i.e. mainly depending on the species from which the erythrocyte originates). The lowest porosity limit for the filtration membrane is the diameter of the involved free cell from a species; the diameter of a human RBC is 6 to 8 µm. Included in the porosity range is two times the diameter of the involved free cell. If a single IgM is involved, a pentamerous hemagglutination has a diameter of about 16 µm. In practice, multiple IgM are involved and thus the diameter will be bigger than 16 µm.

Preferably, the filtration membrane is fixed to the inside of the test unit 1 by means of a seal ring (i.e. washer) such as rubber. Alternatively, the filtration membrane can be glued or welded to the inside of a test unit. Welding can be done by thermal, ultrasonic, laser or radiofrequency sealing process. The assembly can also be performed by using a suitable glue and /or solvent bonding. The separation means or the filtration membrane might be formed in continuity with the readable device by 3D printing for instance and thereby form an integral part of the readable device.

In a preferred embodiment, the platform of the readable device comprises at least one transparent area in which the visual blood volume indication means is positioned. The transparent area covers 5% to 99%, preferably 10% to 90%, more preferably 20% to 80%, even more preferably 30% to 70%, most preferably 40% to 60% of the readable device surface. Preferably, the device comprises at least one air vent. More preferably, at least one air vent is provided in each test unit of the device.

In a preferred embodiment, the readable device comprises at least one legible identification means in which information is encoded, said information is selected from the group comprising the type of the readable device, the date of manufacture, the date of validity of the readable device or any combination thereof. The identification means can be an RFID, a bar code, or any other means known to the skilled person.

The readable device can be of any shape and preferably has a disk shape. Preferably, said disk is provided with a central opening 4. The diameter of said disk is from 5 to 20 cm, preferably of from 6 to 15 cm, more preferably of from 7 to 12 cm, most preferably of from 8 to 10 cm. The device might be a single use device or a reusable device.

In a preferred embodiment, the reading device comprises at least one user interface 21 which is connected to any element of the reading device. Said user interface might comprise at least one screen on which information might be displayed to the user. The screen might be a touch screen which allows displaying information and/or allows the user to introduce information or instruct the reading device. The user interface might also comprise at least one screen as described above and/or at least one vocal interface.

For example, but not restricted to, the user interface allows the user to review data previously collected by the device, to check the presence and specifications of a readable device, to verify the correct placement of the reading device on a surface, to verify the correct placement of the readable device into the reading device, to lock and unlock the device, to manage the battery status, et cetera.

In a preferred embodiment, the housing is designed and/or dimensioned to receive a readable device according to any embodiment of the invention. Preferably, the housing comprises at least one protruding central means at which the central opening of the readable device is inserted.

The recognition means is designed to recognize the identification means of the readable device according to any embodiment of the invention. Preferably, the recognition means reads the identification means of the readable device automatically after closing the lid of the device. The recognition means is preferably connected to a signaling means for providing the user with an informative signal as to the presence and/or the compatibility of the readable device with the reading device, said signaling means is a visual and/or an auditory means. Said informative signal might be auditory and/or visual and is:
- a positive signal if a readable device according to any embodiment of the invention is properly inserted in the housing of the reading device,
- a negative signal if a readable device according to any embodiment of the invention is improperly inserted in the housing of the reading device,
- a negative signal if a different readable is inserted in the housing of the reading device, or
- a negative signal if no readable device is inserted in the housing.

The recognition means of the reading device might also read information relative to the validity of the readable device. Said information is encoded in the identification means of the readable device and/or on a server. This means that when a batch of readable devices is received by a receiving party such as a hospital, said receiving party proceeds to the validation of the full batch. The validated batch of readable devices is then stored for further use and information on its validity is stored in the identification means of the readable devices and/or on a server. When a readable device is to be used, the recognition means of the reading device reads the identification means of the readable device and/or connects to the server for confirming the validity of the readable device. The reading device provides a positive or a negative signal to the user with respect to the validity of the readable device.

The rotational means of the reading device might be automatically activated upon closure of the lid. Said rotational means might also be manually activated through the user's interface of the device or via at least one start/off button 22 provided on the reading device and accessible to the user. Preferably, the lid comprises at least one closable opening through which at least one and preferably only one sample receiving means of the readable device is accessible. The closable opening of the lid allows introducing blood volume into the sample receiving means.
Preferably, the rotational means comprises a stepper motor allowing a configurable rotation in direction and speed, similar to a CD or DVD player.

The reading device allows selection of the appropriate sample receiving means of the readable device by limiting access to a single sample receiving means. Selection of the appropriate sample receiving means is allowed by and comprises reading the electronically legible differentiation means of the readable device. Selection of the appropriate sample receiving means might further comprise reading at least one label positioned on a container or a bag comprising the biological sample. The reading device reads the label in which information on the sample and/or donor is encoded and aligns the appropriate sample receiving means to the closable opening of the lid.

The reading device might be connected to an electric source and/or provided with batteries. Said batteries have autonomy of at least 30 minutes, preferably at least one hour, more preferably at least 2 hours and most preferably at least 3 hours. The batteries are preferably rechargeable in a short time of at most 15 min. Preferably, and if applicable, the battery status is continuously monitored and can be consulted by the user via the user interface. The user is also notified by a visual and/or auditory signal to warn the user of the remaining device autonomy. Preferably, warning signals are produced when the battery charge is below a configurable level and a critical level, in order to notify the user. Preferably, when the critical threshold is exceeded, an automatic shutdown procedure is started. If an analysis was in progress, the device will shut down as soon as the analysis is finished.

In a preferred embodiment, the reading device comprises at least one recording means for recording information selected from identity of the user, identity of the tested samples and/or of the donor, number of tests per day, the results of each test or any combination thereof. Preferably, the reading device also comprises at least one identification means.

In a preferred embodiment, the reading device comprises at least one connection means for connecting the device to at least one lab info system (LIS), at least one Ethernet network connection, at least one internet network, at least one wireless connection means, at least one printer, at least one scanner or any combination thereof. Preferably, the reading device further comprises at least one connection means for connecting at least one USB temporary storage means. The reading device might be connectable to a docking station through which the above mentioned connections are provided.

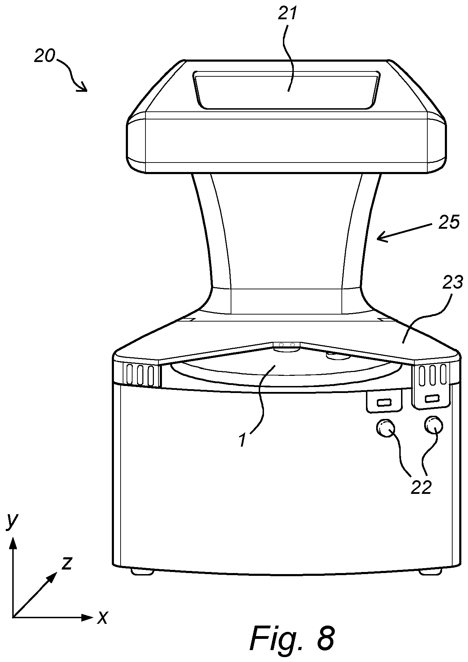

In a preferred embodiment, the height of the reading device is of from 0.1 to 0.5 m, preferably from 0.15 to 0.4 m, more preferably from 0.2 to 0.3 m, most preferably about 0.25 m. The width of the reading device is of from 0.05 to 0.3 m, preferably from 0.1 to 0.25 m, more preferably from 0.15 to 0.2 m. The length of the reading device is of from 0.05 to 0.3 m, preferably from 0.1 to 0.25 m, more preferably from 0.15 to 0.2 m. The height, the width and the length are respectively measured according to the "Y", "Z" and "X" directions shown in figure 8. Preferably, the weight of the reading device is of from 0.5 to 3 kg, preferably from 1 to 2.5 kg, more preferably from 1.5 to 2 kg, most preferably about 1.7 kg. The dimensions and the weight of the reading device provide an easy transport to the user. In a preferred embodiment, the reading device comprises at least one grip element (25 in figure 8) through which the device can be transported or moved.

In a preferred embodiment, the base of the reading device is designed is such a way that stability on a flat surface is ensured. More in particular, the base of the reading device is provided with support rubber knobs which prevent the device from shoving and minimize possible external vibrational disturbances such as other equipment present in the direct vicinity of the reading device.

In a preferred embodiment, the housing material of the reading device must comply with UL 94-V0, the standard for safety of flammability of plastic materials for parts in devices and appliances testing, in which is stated that burning stops within 10 seconds on a vertical specimen and drips of particles are allowed as long as they are not inflamed. Preferably, the housing material is easy to clean and resistant to common chemical disinfectants such as isopropyl alcohol and bleach.

In a preferred embodiment, the disk inlet cover must meet the IP54 protection standard which states that the inlet has limited protection against dust ingress and is protected against splash water from any direction.

In a preferred embodiment, the reading device must comply with IEC 61010-2-020, which comprises the safety requirements for electrical equipment for measurement, control, and laboratory use, in particular electrically powered laboratory centrifuges.

In a preferred embodiment, the reading device comprises an angle sensor. The angle sensor measures the relation by which any position with respect to any other position is established. It calculates the orientation of the device with respect to a specified reference position as expressed by the amount of rotation necessary to change from one orientation to the other about a specified axis. The implementation of an angle sensor allows the correct placement of the reading device by informing the user about position of the device via the user interface and visual and/or auditory signals.

In a preferred embodiment, the reading device comprises a first optical sensor which is a diode couple or fixed sensor that will transmit a signal to the processor. Said first optical sensor detects the presence of at least one binding pair of molecules in the separation means of the readable device and/or the presence of free molecules in the detection chamber of the readable device.

In a preferred embodiment, the reading device comprises a second optical sensor which is a diode couple or fixed sensor that will transmit a signal to the processor. Said second optical sensor detects the presence of a liquid biological sample introduced in the sample receiving means.

In a preferred embodiment, the rotational means is allowed to switch movements at predetermined timestamps, including but not restricted to a continuous rotation, a shake motion and, a wave motion. The continuous rotation is a unidirectional movement at a set rotation speed. The shake motion can be defined as a bidirectional rotational movement in which the movement is alternated in both directions, yielding a shake effect. The wave motion is a unidirectional movement with a speed variating according to a wave function. Preferably, the continuous rotational movement can further be characterized by parameters which include, but are not restricted to, the direction of rotation, the starting rotation speed, the stable rotation speed, the cycle time, and the acceleration. Preferably, the shake motion can further be characterized by parameters which include, but are not restricted to, the number of shakes, the speed values of each shakes, the completion time of a cycle, and the acceleration.

In a preferred embodiment, the reading device comprises a data storage unit and a processor. Depending on the processor, a heat sink can be integrated.

In a third aspect, the present invention provides a method for performing immunoassay tests comprising the steps of:
a) introducing at least one liquid biological sample in the sample receiving means of a readable device according to any embodiment of the invention,
b) inserting the readable device in the housing 24 of a reading device 20 according to any embodiment of the invention, steps (a) and (b) being interchangeable,
c) flowing the liquid biological sample in the test units by rotating the readable device by the rotation means of the reading device 20, and
d) determining the result of the immunoassay test by the first optical sensor of the reading device 20, said first optical sensor detects the presence of at least one binding pair of molecules in the separation means of the readable device and/or the presence of free molecules in the detection chamber of the readable device,
wherein the volume of the liquid biological sample introduced in the sample receiving means is detected by:
- the visual volume indication means of the readable device, and/or
- the electronically legible volume indication means of the readable device and the second optical sensor of the reading device 20.
Preferably, when step (b) is performed before step (a), the blood samples are introduced in the sample receiving means of the readable device through the closable opening of the lid of the reading device.

It is to be understood that rotation speeds of the reading device might be constant or variable through the different steps of the method. Said rotation speeds might be adapted to accomplish the different steps of the method. It is also to be understood that rotation of the readable device might be stopped at any time of the method and after or before any step of said method.

In a preferred embodiment, the method comprises the step of rotating the readable device at a predetermined speed which is sufficient to flow the blood through the first blocking means of the outlet of the receiving chambers. Said predetermined speed is comprised between 1 and 3000 rotation per minute (rpm). Then, rotation is maintained at the same predetermined speed or different speed thereby flowing the blood into the distribution chambers of the test units. The rotation might be:
- maintained at a predetermined speed which is sufficient to flow the blood through the blocking means 9 of the outlets of the distribution chambers. Said predetermined speed is comprised between 1 and 3000 rpm. The rotation is maintained at the same or different speed thereby flowing the blood into the reagent incubation chamber, through the separation means to finally rest in the detection chamber. The first optical sensor of the reading device detects the presence of hemagglutinated erythrocytes in the separation means of the readable device and/or the presence of free erythrocytes in the detection chamber of the readable device thereby determining the blood group. Preferably the result of the test and/or the determined blood group is shown on the user interface of the reading device and/or recorded by the recording means of the same device. The second optical sensor of the reading device detects the presence of blood in the overflow chambers of the readable device and provides the user with information on the presence of blood in said chambers.
- stopped and the overflow chambers of the readable device are optically read by the second optical sensor of the reading device. If blood is present in said overflow chambers, a positive signal is provided to the rotation means of the reading device to continue the test. If no blood is present in the at least one of the overflow chambers, the reading device provides instruction to the user to perform the test a second time and/or aligns the sample receiving means having insufficient blood volume with the closable opening of the reading device and requests the user to add sufficient blood volume in order to continue the test as described above.

In a preferred embodiment, the time required for running the test using the method and/or the readable device and/or the reading device of the present invention is of from 5 seconds to 5 min, preferably from 30 seconds to 4 min, preferably from 1 min to 3 min, most preferably from 1.5 min to 2 min. The invention provides quick, efficient and reliable blood group determination thereby avoiding erroneous blood transfusions and meeting the urgent needs in hospitals for urgent intervention for instance. The devices and/or the method of the invention might also be used outside the hospital such as on army fields or by non-governmental organizations.

In a preferred embodiment, the different steps of the method are contained in at least one algorithm which is implemented in the reading device. Said algorithm detects the presence of a readable device according to an embodiment of the invention and/or provides instructions and/or information to the user. It is to be understood that any step or action described above might be contained in the algorithm of the reading device. In a preferred embodiment, the algorithm further comprises the different predetermined speeds at which the readable device is rotated for accomplishing each step of the method. In a preferred embodiment, the algorithm further comprises the different predetermined running times for each step of the method.

In a possible embodiment, the method compromises an additional step of comparing data, which is contained in at least one algorithm, which is implemented in the reading device. Said algorithm compares two or more datasets and provides instructions and/or information to the user. In a preferred embodiment, the data to be compared can either be internal data, external data, or a combination of the two. The internal data can be obtained from the storage unit incorporated in the reading device. The external data can be obtained through one or more connection means from one or more lab info systems (LIS), servers, and/or external storage units. In a preferred embodiment,

In a preferred embodiment, the method further comprises the step of providing a message to the user, said message comprises information on the detection of the blood sample volume by the second optical sensor of the reading device.

In another aspect, the present invention provides a kit comprising at least one readable device as described above and/or a reading device as described above and/or at least one leaflet comprising instructions to the user.

It is supposed that the present invention is not restricted to any form of realization described previously and that some modifications can be added to the presented example without reappraisal of the appended claims.

The invention is further described by the following non-limiting examples which further illustrate the invention, and are not intended to, nor should they be interpreted to, limit the scope of the invention.

### EXAMPLE

The present invention will now be further exemplified with reference to the following examples. The present invention is in no way limited to the given examples.

If necessary, the reading device is also able to work offline (without LIS). In this case, there are two options:
- permanently offline: the device is not used in a structure equipped with an LIS
- temporary offline: to compensate for a lack of time (such as a medical emergency) or a temporary disconnection to the local network (LIS)

The reading device houses a data storage unit to store the results of the test locally. In the case of a use with LIS, the machine can work in two modes, exemplified by example 1 and 2. In the case of a use with or without LIS (permanent or temporary), the machine can work in the same two modes, transfusion mode (example 1 and 3) and lab mode (example 2 and 4). Take note however that if possible, it is recommended to use the reading device while connected to LIS, since this allows an additional level of security.

The reading device can be programmed by the administrator for several cases:
- The reading device can only be used with LIS (standard and recommended);
- The reading device can be used with or without LIS;
- The reading device can only be used without LIS.

The reading device can also be used to check the compatibility at the level of platelets and plasma. To find out if a blood bag contains red blood cells or platelets or plasma, it is necessary to refer to the bar code(s) present on the blood bag. An example of platelets/plasma testing is given in example 5.

### Example 1: Transfusion Mode Sequence (with LIS)

### Data recovery & Start up

- The transfusion prescriptions are generated.
- The user turns on the reading device when still on its charging base.
- The user logs in in via the user interface.
- The user scans the badge of the patient considered for transfusion.
- The reading device queries the hospital's database.
   ∘ If there is no transfusion scheduled for this patient, the user is notified by a message and the test is stopped.
   ∘ Scheduled transfusions → The list (1) of blood bags associated with the patient is downloaded on the reading device
   ∘ List (2) of available readable devices is downloaded onto the reading device
- The user can repeat this operation several times (by scanning the patient's badge).
- When the reading device has downloaded all the information, the user can (if the battery charge allows it) take the reading device from its charging base to start the analysis.

### Testing

- The user identifies and validates the readable device (validated at the central lab / expiry date) or the bag containing the readable device.
- The following varies according to the analysis purpose of the reading device:

### Comparing a patient's blood with the blood of a blood bag

- The user identifies the patient (by scanning his/her badge) and the blood bag to be transfused.
   ∘ If the patient is not in the imported list, a message indicates that the patient was not found and the test is stopped.
   ∘ If the blood bag is not in the imported list, a message indicates that the blood bag was not found and the test is stopped
   ∘ If the blood bag cannot be transfused to the patient, a message indicates that the patient and blood bag are incompatible and the test is stopped.
- The user injects the patient's blood in one side and the blood of the patient's blood bag in the other side of the readable device.
- The user inserts the readable device into the reading device
- As soon as the lid is closed, the reading device identifies the readable device and check the readable device by comparing it with the readable device list (2)
   ∘ If the readable device is not in the list (not validated at the central lab / expiry date exceeded / wrong type of readable device), the user is notified by a message and the test is stopped.
- Once the readable device is recognized, the reading device offers the user a last chance to interrupt the analysis. After 15 seconds, the analysis starts automatically.
- When the reading device is executing the centrifugation process, the lid is locked. If an error occurs, the centrifugation process must be stopped before opening the lid.
- When the centrifugation process is finished, the reading device yields a result of compatibility.
   ∘ If the blood from the blood bag cannot be transfused to the patient, the user is notified by a message and the test is stopped.
- Once the compatibility has been established, the transfusion can start

### Comparing the blood of a first blood bag with the blood of a second blood bag

- The user identifies the patient and the blood bags considered for transfusion.
   ∘ If the patient is not in the imported list, a message indicates that the patient was not found and the test is stopped.
   ∘ If one or both blood bags are not in the imported list, a message indicates that the blood bags were not found and the test is stopped.
   ∘ If one or both blood bags cannot be transfused to the patient a message indicates that the patient and the blood bags are incompatible and the test is stopped.
- The user injects the blood from the first pocket in one side and the one from the second in the other side of the readable device.
- The user inserts the readable device into the reading device.
- As soon as the lid is closed, the reading device identifies the readable device and check the readable device by comparing it with the readable device list (2)
   ∘ If the readable device is not in the list (not validated at the central lab / expiry date exceeded / wrong type of readable device), the user is notified by a message and the test is stopped.
- Once the readable device is recognized, the reading device offers the user a last chance to interrupt the analysis. After 15 seconds, the analysis starts automatically.
- When the reading device is executing the centrifugation process, the lid is locked. If an error occurs, the centrifugation process must be stopped before opening the lid.
- When the centrifugation process is finished, the reading device yields a result of compatibility.
   ∘ If the blood from one of the blood bags cannot be transfused to the patient, the user is notified and the test is stopped.
- Once the compatibility of at least one of the blood bags has been established, the transfusion can start.

### Transfer of results & Termination

When all blood bags associated with the patient are transfused the user can put the reading device back on its charging base. The reading device shall then transfer the gathered results to LIS. While on the charging base, the user can also scan a new patient badge in order to download a new list of transfusions to perform.

While transfusions are in progress, it is possible to put the reading device back onto its charging base in order to download a new list of transfusions to be performed. In this case, only results related to a patient for whom all transfusions have been performed are returned to the DSL.
Furthermore, blood bag analysis is only possible if a patient-blood bag analysis was previously performed on the same reading device and in the meantime the results for this patient have not been returned to the LIS.

### Example 2: Lab Mode Sequence (with LIS)

In this case, a patient-blood bag readable device is used and only the cavity on the "patient side" of the readable device is used. The device is not designed to test a blood bag in the lab.

### Data recovery & Start up

- The test requirements are generated.
- The user turns on the readable device while on its charging base.
- The user logs in via the user interface.
- The user scans the badge of the patient for which he wishes to test the blood group.
- The reading device queries the hospital's database.
   ∘ If there is no transfusion scheduled for this patient, the user is notified by a message and the test is stopped.
   ∘ Scheduled transfusions → The list (1) of blood bags associated with the patient is downloaded on the reading device
   ∘ List (2) of available readable devices is downloaded onto the reading device
- The user can repeat this operation several times (by scanning the patient's badge).
- When the reading device has downloaded all the information, the user can (if the battery charge allows it) take the reading device to start the analysis.

### Testing

- The user identifies and validates the readable device (validated at the central lab / expiry date) or the bag containing the readable device.
- The user identifies the patient (badge scan) whose blood type is to be determined.
   ∘ If the patient is not in the imported list, a message indicates that the patient was not found and the test is stopped.
- The user takes the readable device and injects the patient's blood into the cavity on the "patient side' of the readable device.
- The user inserts the disc into the reading device
- As soon as the lid is closed, the reading device identifies the readable device and checks by comparison with the readable device list (2).
   ∘ If the readable device is not in the list (not validated at the central office / expiry date exceeded /wrong type), the user is notified by a message and the test is stopped.
- Once the readable device is recognized, the reading device offers the user a last chance to interrupt the analysis. After 15 seconds, the analysis starts automatically.
- When the reading device is executing the centrifugation process, the lid is locked. If an error occurs, the centrifugation process must be stopped before opening the lid.
- When the centrifugation process is finished, the reading device yields the blood group of the blood tested.

### Transfer of results & Termination

When all tests are done, the user puts the reading device back on its charging base. The reading device transfers the results to the LIS and downloads a new list of tests to be performed if another patient's badge has been scanned.

### Example 3: Transfusion Mode Sequence (without LIS)

### Start-up

- The user turns on the reading device.
- The user logs in in via the user interface.
- The user scans the badge of the patient considered for transfusion.

### Testing

- The user identifies and validates the readable device (expiry date) or the bag containing the readable device.
- The following varies according to the analysis purpose of the reading device:

### Comparing a patient's blood with the blood of a blood bag

- The user identifies the patient (by scanning his/her badge) and the blood bag to be transfused.
   ∘ If there is temporarily no connection to LIS, a message indicates that the patient was not found and asks the user if he wants to continue. If there is permanently no connection to LIS, there is no message warning.
   ∘ If there is temporarily no connection to LIS, a message indicates that the blood bag was not found and asks the user if he wants to continue. If there is permanently no connection to LIS, there is no message warning.
- The user injects the patient's blood in one side and the blood of the patient's blood bag in the other side of the readable device.
- The user inserts the readable device into the reading device.
- As soon as the lid is closed, the reading device identifies the readable device and checks the readable device (expiry date exceeded / wrong type of readable device)
- Once the readable device is recognized, the reading device offers the user a last chance to interrupt the analysis. After 15 seconds, the analysis starts automatically.
- When the reading device is executing the centrifugation process, the lid is locked. If an error occurs, the centrifugation process must be stopped before opening the lid.
- When the centrifugation process is finished, the reading device yields a result of compatibility.
   ∘ If the blood from the blood bag cannot be transfused to the patient, the user is notified by a message and the test is stopped.
- Once the compatibility has been established, the transfusion can start.

### Comparing the blood of a first blood bag with the blood of a second blood bag

- The user identifies the patient and the blood bags considered for transfusion.
   ∘ If the blood group of the patient has not yet been validated, the user is notified by a message and the test is stopped.
- The user injects the blood from the first pocket in one side and the one from the second in the other side of the readable device.
- The user inserts the readable device into the reading device.
- As soon as the lid is closed, the reading device identifies the readable device and checks its expiry date as well as his type.
   ∘ If the readable device is faulty (expiry date exceeded /wrong type), the user is notified by a message and the test is stopped.
- Once the readable device is recognized, the reading device offers the user a last chance to interrupt the analysis. After 15 seconds, the analysis starts automatically.
- When the reading device is executing the centrifugation process, the lid is locked. If an error occurs, the centrifugation process must be stopped before opening the lid.
- When the centrifugation process is finished, the reading device yields a result of compatibility.
   ∘ If the blood from one of the blood bags cannot be transfused to the patient, the user is notified by a message and the test is stopped.
- Once the compatibility of at least one of the blood bags has been established, the transfusion can start.

In the case of a permanent use without LIS, a maintenance technician will have to come to recover the data regularly to prevent the disk from being saturated. Before each analysis, the reading device automatically checks if there is enough space to store the results.

### Example 4: Lab Mode Sequence (without LIS)

In this case, a patient-blood bag readable device is used and only the cavity on the "patient side" of the readable device is used. The device is not designed to test a blood bag in the lab.

### Data recovery & Start up

- The test requirements are generated.
- The user turns on the readable device while on its charging base.
- The user logs in via the user interface.
- The user scans the badge of the patient for which he wishes to test the blood group.
- The reading device queries the hospital's database.
   ∘ If there is no transfusion scheduled for this patient, the user is notified by a message and the test is stopped.
   ∘ Scheduled transfusions → The list (1) of blood bags associated with the patient is downloaded on the reading device
   ∘ List (2) of available readable devices is downloaded onto the reading device
- The user can repeat this operation several times (by scanning the patient's badge).
- When the reading device has downloaded all the information, the user can (if the battery charge allows it) take the reading device to start the analysis.

### Testing

- The user identifies and validates the readable device (validated at the central lab / expiry date) or the bag containing the readable device.
- The user identifies the patient (badge scan) whose blood type is to be determined.
   ∘ If there is no temporary connection to LIS, a message indicates that the patient was not found and asks the user if he wants to continue. If there is no permanent connection, there is no message warning.
- The user takes the readable device and injects the patient's blood into the cavity on the "patient side' of the readable device.
- The user inserts the disc into the reading device
- As soon as the lid is closed, the reading device identifies the readable device and checks its expiry date as well as his type.
   ∘ If the readable device is faulty (expiry date exceeded /wrong type), the user is notified by a message and the test is stopped.
- Once the readable device is recognized, the reading device offers the user a last chance to interrupt the analysis. After 15 seconds, the analysis starts automatically.
- When the reading device is executing the centrifugation process, the lid is locked. If an error occurs, the centrifugation process must be stopped before opening the lid.
- When the centrifugation process is finished, the reading device yields the blood group of the blood tested.

### Termination

When all tests are done, the user puts the reading device back on its charging base.

### Example 5: Plasma/Platelets Sequence (with and without LIS)

For plasma or platelet sequences, only the transfusion mode can be used. In this case, we apply the sequence described in Example 1 by replacing the "Testing" part by the following sequence:

### Testing:

- The user identifies the patient (by scanning his/her badge) and the blood bag to be transfused.
   ∘ If there is a connection to LIS, and the patient is not in the imported list, a message indicates that the patient was not found and the test is stopped. If there is temporarily no connection to LIS, a message indicates that the patient was not found and asks the user if he wants to continue. If there is permanently no connection to LIS, there is no message warning.
   ∘ If there is a connection to LIS, and the blood bag is not in the imported list, a message indicates that the blood bag was not found and the test is stopped. If there is temporarily no connection to LIS, a message indicates that the blood bag was not found and asks the user if he wants to continue. If there is permanently no connection to LIS, there is no message warning.
- If the product code corresponds to plasma or platelets, the reading device indicates that the patient and the patient blood bag are compatible.

It is also possible to program the reading device in order to bypass the blood group test using the reading device, only validating if the blood bags were allocated to patients (for example in the case of prematurely newborn infants).

### Example 6: Transfusion Mode Test (with LIS)

Example 6 describes a bedside test based on the sequence proposed in Example 1. The blood of a patient is compared with that of a blood bag in order to prepare for a blood transfusion, using the transfusion mode (with connection to LIS). This process is subdivided in several detailed steps.

The user starts up the reading device still on its charging base. A test sequence is automatically run by the reading device to test the following features:
- The presence of all the files necessary for the proper functioning.
- The communication between the equipment and the external network (LIS) if based on load and communication.
- The proper functioning of the engine and associated sensors.
- The charge of the battery.
- The intensity of the source LED and associated sensor.
- The intensity of the LED / photodiode internal circumferential identifier.
- The smooth operation of the barcode reader.
When the self-test is performed, a message is generated to transmit to the system that the equipment is in good working order or not (error clearly identified). Following a message that the equipment is in good working order, the user can log in via the user interface.

Using the reading device, the user scans the badge of the patient considered for transfusion. Meanwhile, the reading device queries and downloads the hospital's database, including the patient list and the readable list. Here, a first check is executed:
- If there is no transfusion scheduled for this patient, the user is notified of this via a message and the test is stopped.
- If there is a transfusion scheduled for this patient, a list of blood bags associated with the patient as well as a list of readable devices is downloaded on the reading device.

Using the reading device, the user scans the bar code on the blood bag:
- If the scanned blood bag does not match a blood bag associated with the list of the patient, the user is notified of this via a message.
- If the scanned blood bag is on the list of the patient, the device may be removed from its charging base to start the analysis.

First, blood sampling is executed. The 2 wells on the readable device receive blood samples from respectively a patient and a blood bag. After opening the lid of the reading device, the readable device is inserted into the reading device.

From each of the aforementioned wells, 4 microfluidic channels allow the blood sample to separate into 4 subsamples which by centrifugation will move to the incubation chambers downstream, each containing a reagent. The four reagents containing the antibodies and a corresponding control buffer determine the 3 blood groups and the Rh D.

The principle at the base of this test is the haemagglutination principle, which is a specific form of agglutination that involves red blood cells (RBCs). RBCs that carry a certain antigen, will be recognized by the antibodies in the reagent if those antibodies bind the antigens. This causes the agglutination of the RBCs, producing a blood clump that will be retained by a filter located downstream in the circuit microfluidics. On the other hand, this filter lets pass the individual RBCs that are not agglutinated (exempli gratia if they do not carry the antigen recognized by the corresponding antibody A, B or D). After the filter, a cavity for receiving the non-agglutinated portion of the sample serves as a window for an optical quantification system. These viewing windows are at the edge of the disc. The cavities are configured so to optimize the detection of non-agglutinated RBCs by a diode detection system. The detection zone is of the order of 1.5 to 2 mm². A transceiver pair (photodiode) will allow the measurement in opacity on the zone of detection. The measurement is insensitive to color and therefore well determined with respect to opacity. Due to the rotation of the disc, a continuous reading can be performed. When a reading window goes into the detection system, a peak of which the height is proportional to the quantity of RBCs detected, appears on a display graph of the signal. If the height of the peak (or the area under the curve) is small, haemagglutination has occurred. If the height of the peak is large, the entire sample has passed through the filter, and therefore no agglutination reaction has occurred. By comparing the value with a reference value, the intensity of the signal makes it possible to determine the antigen-antibody reaction, which in turn allows the validation of the blood group of the sample. Thanks to a symmetry, the disc allows to receive 2 samples and thus to carry out two tests in parallel. The 2 results generated can therefore be compared by software integrated into the equipment to check the compatibility of the patient's blood with the blood bag.

The strength of this test lies within the "double" blood group verification at bedside, through a first check (comparing the blood bag data with the patient data through bar code and badge scanning) and a second check (blood testing on the readable device). This double verification allows the prevention of almost 100% of the ABO incompatibility complications at bedside.

## Claims

1. A device for performing immunoassay tests comprising:
a. a readable device (1) for performing immunoassay tests comprising a platform which comprises at least two sample receiving means (2, 3, 2', 3') for receiving at least one liquid biological sample from at least one donor and at least one donor differentiation means for differentiating different donor types; each sample receiving means is fluidly connectable to at least two test units, each of said test units comprises at least one reagent incubation chamber (12) which is fluidly connected to at least one separation means, said separation means is fluidly connected to at least one detection chamber (13), **characterized in that** the platform comprises at least one volume indication means for detecting the presence of a predetermined volume of the liquid biological sample.
b. a reading device (20) for performing immunoassay tests comprising at least one housing (24) in which an aforementioned readable device is insertable, said housing (24) is connected to at least one rotational means for rotating the readable device at a predetermined rotation speed; a lid (23) movable between an open position in which the readable device is visible and a closed position in which the readable device is not visible; at least one recognition means for recognizing the presence of the readable device and/or for extracting information from the legible identification means of the readable device; at least one first optical sensor which detects the presence of at least one binding pair of molecules in the separation means of the readable device and/or the presence of free molecules in the detection chamber of the readable device, and; at least one second optical sensor which detects the presence of a liquid biological sample in the overflow chamber of the readable device.

2. The readable device according to claim 1 wherein the donor differentiation means is a visual means (X, Y) which is visible to the user and/or an electronically legible differentiation means which is readable by a suitable reading device (20).

3. The readable device according to any of claims 1 or 2 wherein the volume indication means is a visual means (5) which is visible to the user and/or an electronically legible volume indication means which is readable by a suitable reading device (20).

4. The readable device according to any of claims 1 to 3 wherein the electronically legible volume indication means comprises at least one overflow chamber (10) which is fluidly connectable to the sample receiving means (2, 3, 2', 3').

5. The readable device according to any of claims 1 to 4 wherein the sample receiving means have a shape that protrudes (2, 3) from the platform and/or a shape devoid of protrusion (2', 3') from the platform.

6. The readable device according to any of claims 1 to 5 wherein the sample receiving means has a shape selected from the group comprising a tube, a tube comprising at least one liquid biological sampling means, an open cavity in which the liquid biological sample can be deposited or an open cavity comprising at least one liquid biological sampling means.

7. The readable device according to any of claims 1 to 6 wherein the platform comprises at least one transparent area (14) in which the visual volume indication means is positioned.

8. The readable device according to any of claims 1 to 7 comprising at least one legible identification means in which information is encoded, said information is selected from the group comprising the type of the readable device, the date of manufacture, the date of validity of the readable device or any combination thereof.

9. The reading device (20) according to claim 1 wherein the recognition means is connected to a signaling means for providing the user with an informative signal as to the presence and/or the compatibility of the readable device with the reading device (20), said signaling means is a visual and/or an auditory means.

10. The reading device (20) according to claim 9 wherein the lid (23) comprises at least one closable opening through which at least one sample receiving means of the readable device is accessible.

11. The reading device (20) according to any of claims 9 to 10 comprising at least one user interface (21) which is connected to any element of the reading device (20), said interface provides and/or receives information from the user, said information is selected from the group comprising select a rotation speed program, start rotation, show results in readable form, show error message.

12. A method for performing immunoassay tests comprising the steps of:
a) introducing at least one liquid biological sample in the sample receiving means of a readable device as described in any of claims 1 to 8,
b) inserting the readable device in the housing (24) of a reading device (20) as described in claim 1 and in any of claims 9 to 11, steps (a) and (b) being interchangeable,
c) flowing the liquid biological sample in the test units by rotating the readable device by the rotation means of the reading device (20), and
d) determining the result of the immunoassay test by the first optical sensor of the reading device (20), said first optical sensor detects presence of at least one binding pair of molecules in the separation means of the readable device and/or the presence of free molecules in the detection chamber of the readable device,
wherein the volume of the liquid biological sample introduced in the sample receiving means is detected by:
- the visual volume indication means of the readable device, and/or
- the electronically legible volume indication means of the readable device and the second optical sensor of the reading device (20).

13. The method according to claim 12 wherein when step (b) is performed before step (a), the liquid biological sample are introduced in the sample receiving means of the readable device through the closable opening of the lid (23) of the reading device (20).

14. The method according to any of claims 12 or 13 further comprising the step of providing a message to the user, said message comprises information on the detection of the liquid biological sample volume by the second optical sensor of the reading device (20).

## Patentansprüche

1. Vorrichtung zum Ausführen von Immunassay-Tests, Folgendes umfassend:
a. eine ablesbare Vorrichtung (1) zum Ausführen von Immunassay-Tests, eine Plattform umfassend, die mindestens zwei Probenaufnahmemittel (2, 3, 2', 3') zum Aufnehmen von mindestens einer flüssigen biologischen Probe von mindestens einem Spender und mindestens ein Spenderunterscheidungsmittel zum Unterscheiden verschiedener Spenderarten umfasst, wobei jedes Probenaufnahmemittel in Fluidverbindung mit mindestens zwei Testeinheiten gebracht werden kann, wobei jede der Testeinheiten mindestens eine Reagenzinkubationskammer (12) umfasst, die in Fluidverbindung mit mindestens einem Trennmittel steht, wobei das Trennmittel in Fluidverbindung mit mindestens einer Detektionskammer (13) steht, **dadurch gekennzeichnet, dass** die Plattform mindestens ein Volumenanzeigemittel zum Detektieren des Vorhandenseins eines festgelegten Volumens der flüssigen biologischen Probe umfasst,
b. eine Lesevorrichtung (20) zum Ausführen von Immunassay-Tests, mindestens ein Gehäuse (24) umfassend, in das eine zuvor genannte ablesbare Vorrichtung einsetzbar ist, wobei das Gehäuse (24) mit mindestens einem Drehmittel zum Drehen der ablesbaren Vorrichtung mit einer festgelegten Drehgeschwindigkeit verbunden ist, einen Deckel (23), der zwischen einer geöffneten Position, in der die ablesbare Vorrichtung sichtbar ist, und einer geschlossenen Position, in der die ablesbare Vorrichtung nicht sichtbar ist, beweglich ist, mindestens ein Erkennungsmittel zum Erkennen des Vorhandenseins der ablesbaren Vorrichtung und/oder zum Extrahieren von Informationen von dem lesbaren Identifikationsmittel der ablesbaren Vorrichtung, mindestens einen ersten optischen Sensor, der das Vorhandensein von mindestens einem Bindungsmolekülpaar in dem Trennmittel der ablesbaren Vorrichtung und/oder das Vorhandensein von freien Molekülen in der Detektionskammer der ablesbaren Vorrichtung detektiert, und mindestens einen zweiten optischen Sensor, der das Vorhandensein einer flüssigen biologischen Probe in der Überlaufkammer der ablesbaren Vorrichtung detektiert.

2. Ablesbare Vorrichtung nach Anspruch 1, wobei das Spenderunterscheidungsmittel ein visuelles Mittel (X, Y) ist, das für den Benutzer sichtbar ist, und/oder ein elektronisch lesbares Unterscheidungsmittel, das durch eine geeignete Lesevorrichtung (20) ablesbar ist.

3. Ablesbare Vorrichtung nach einem der Ansprüche 1 oder 2, wobei das Volumenanzeigemittel ein visuelles Mittel (5) ist, das für den Benutzer sichtbar ist, und/oder ein elektronisch lesbares Volumenanzeigemittel, das durch eine geeignete Lesevorrichtung (20) ablesbar ist.

4. Ablesbare Vorrichtung nach einem der Ansprüche 1 bis 3, wobei das elektronisch lesbare Volumenanzeigemittel mindestens eine Überlaufkammer (10) umfasst, die in Fluidverbindung mit den Probenaufnahmemitteln (2, 3, 2', 3') gebracht werden kann.

5. Ablesbare Vorrichtung nach einem der Ansprüche 1 bis 4, wobei die Probenaufnahmemittel eine Form aufweisen, die von der Plattform hervorsteht (2, 3), und/oder eine Form, die frei von Vorsprüngen (2', 3') von der Plattform ist.

6. Ablesbare Vorrichtung nach einem der Ansprüche 1 bis 5, wobei das Probenaufnahmemittel eine Form aufweist, die aus der Gruppe ausgewählt ist, die ein Rohr, ein Rohr, das mindestens ein flüssiges biologisches Beprobungsmittel umfasst, einen offenen Hohlraum, in den die flüssige biologische Probe eingebracht werden kann, oder einen offenen Hohlraum, der mindestens ein flüssiges biologisches Beprobungsmittel umfasst, umfasst.

7. Ablesbare Vorrichtung nach einem der Ansprüche 1 bis 6, wobei die Plattform mindestens einen transparenten Bereich (14) umfasst, in dem das visuelle Volumenanzeigemittel positioniert ist.

8. Ablesbare Vorrichtung nach einem der Ansprüche 1 bis 7, mindestens ein lesbares Identifikationsmittel umfassend, in das Informationen codiert sind, wobei die Informationen aus der Gruppe ausgewählt sind, welche die Art der ablesbaren Vorrichtung, das Herstellungsdatum, das Datum der Gültigkeit der ablesbaren Vorrichtung oder eine Kombination daraus umfasst.

9. Lesevorrichtung (20) nach Anspruch 1, wobei das Erkennungsmittel mit einem Signalgebungsmittel verbunden ist, um für den Benutzer ein informatives Signal über das Vorhandensein und/oder die Kompatibilität der ablesbaren Vorrichtung mit der Lesevorrichtung (20) bereitzustellen, wobei das Signalgebungsmittel ein visuelles und/oder ein auditives Mittel ist.

10. Lesevorrichtung (20) nach Anspruch 9, wobei der Deckel (23) mindestens eine verschließbare Öffnung umfasst, durch welche mindestens ein Probenaufnahmemittel der ablesbaren Vorrichtung zugänglich ist.

11. Lesevorrichtung (20) nach einem der Ansprüche 9 bis 10, mindestens eine Benutzerschnittstelle (21) umfassend, die mit einem beliebigen Element der Lesevorrichtung (20) verbunden ist, wobei die Schnittstelle Informationen bereitstellt und/oder vom Benutzer empfängt, wobei die Informationen aus der Gruppe ausgewählt sind, die Drehgeschwindigkeitsprogramm auswählen, Drehen starten, Ergebnisse in lesbarer Form anzeigen, Fehlernachricht zeigen umfasst.

12. Verfahren zum Ausführen von Immunassay-Tests, folgende Schritte umfassend:
a) Einführen mindestens einer flüssigen biologischen Probe in das Probenaufnahmemittel einer ablesbaren Vorrichtung, wie sie in einem der Ansprüche 1 bis 8 beschrieben ist,
b) Einsetzen der ablesbaren Vorrichtung in das Gehäuse (24) einer Lesevorrichtung (20), wie sie in Anspruch 1 und einem der Ansprüche 9 bis 11 beschrieben ist, wobei die Schritte (a) und (b) austauschbar sind,
c) Strömenlassen der flüssigen biologischen Probe in den Testeinheiten durch Drehen der ablesbaren Vorrichtung durch das Drehmittel der Lesevorrichtung (20) und
d) Bestimmen des Ergebnisses des Immunassay-Tests durch den ersten optischen Sensor der Lesevorrichtung (20), wobei der erste optische Sensor das Vorhandensein von mindestens einem Bindungsmolekülpaar in dem Trennmittel der ablesbaren Vorrichtung und/ oder das Vorhandensein von freien Molekülen in der Detektionskammer der ablesbaren Vorrichtung detektiert,
wobei das Volumen der flüssigen biologischen Probe, die in das Probenaufnahmemittel eingeführt wird, durch Folgendes detektiert wird:
- das visuelle Volumenanzeigemittel der ablesbaren Vorrichtung und/ oder
- das elektronisch lesbare Volumenanzeigemittel der ablesbaren Vorrichtung und den zweiten optischen Sensor der Lesevorrichtung (20).

13. Verfahren nach Anspruch 12, wobei, wenn Schritt (b) vor Schritt (a) ausgeführt wird, die flüssige biologische Probe durch die verschließbare Öffnung des Deckels (23) der Lesevorrichtung (20) in das Probenaufnahmemittel der ablesbaren Vorrichtung eingeführt wird.

14. Verfahren nach einem der Ansprüche 12 oder 13, ferner den Schritt des Bereitstellens einer Nachricht für den Benutzer umfassend, wobei die Nachricht Informationen über das Detektieren des Volumens der flüssigen biologischen Probe durch den zweiten optischen Sensor der Lesevorrichtung (20) umfasst.

## Revendications

1. Dispositif de réalisation d'analyses d'immuno-essais comprenant :
a. un dispositif lisible (1) pour la réalisation d'analyses d'immuno-essais comprenant une plateforme qui comprend au moins deux moyens de réception d'échantillon (2, 3, 2', 3') pour la réception d'au moins un échantillon biologique liquide en provenance d'au moins un donneur et au moins un moyen de différenciation de donneurs pour la différenciation de différents types de donneurs ; chaque moyen de réception d'échantillon peut être raccordé de manière fluide à au moins deux unités d'analyse, chacune desdites unités d'analyse comprend au moins une chambre d'incubation de réactif (12) qui est raccordée de manière fluide à au moins un moyen de séparation, ledit moyen de séparation est raccordé de manière fluide à au moins une chambre de détection (13), **caractérisé en ce que** la plateforme comprend au moins un moyen d'indication de volume pour la détection de la présence d'un volume prédéterminé de l'échantillon biologique liquide.
b. un dispositif de lecture (20) pour la réalisation d'analyses d'immuno-essais comprenant au moins un logement (24) dans lequel un dispositif lisible susmentionné est insérable, ledit logement (24) est raccordé à au moins un moyen de rotation pour la rotation du dispositif lisible à une vitesse de rotation prédéterminée ; un couvercle (23) mobile entre une position ouverte dans laquelle le dispositif lisible est visible et une position fermée dans laquelle le dispositif lisible n'est pas visible ; au moins un moyen de reconnaissance pour la reconnaissance de la présence du dispositif lisible et/ou pour l'extraction d'informations à partir du moyen d'identification lisible du dispositif lisible ; au moins un premier capteur optique qui détecte la présence d'au moins une paire de molécules de liaison dans le moyen de séparation du dispositif lisible et/ou la présence de molécules libres dans la chambre de détection du dispositif lisible, et ;
au moins un second capteur optique qui détecte la présence d'un échantillon biologique liquide dans la chambre de débordement du dispositif lisible.

2. Dispositif lisible selon la revendication 1 dans lequel le moyen de différenciation de donneurs est un moyen visuel (X, Y) qui est visible par l'utilisateur et/ou un moyen de différenciation lisible électroniquement qui est lisible par un dispositif de lecture (20) adéquat.

3. Dispositif lisible selon l'une quelconque des revendications 1 ou 2 dans lequel le moyen d'indication de volume est un moyen visuel (5) qui est visible par l'utilisateur et/ou un moyen d'indication de volume lisible électroniquement qui est lisible par un dispositif de lecture (20) adéquat.

4. Dispositif lisible selon l'une quelconque des revendications 1 à 3 dans lequel le moyen d'indication de volume lisible électroniquement comprend au moins une chambre de débordement (10) qui peut être raccordée de manière fluide aux moyens de réception d'échantillon (2, 3, 2', 3').

5. Dispositif lisible selon l'une quelconque des revendications 1 à 4 dans lequel les moyens de réception d'échantillon ont une forme qui fait saillie (2, 3) à partir de la plateforme et/ou une forme dépourvue de saillie (2', 3') à partir de la plateforme.

6. Dispositif lisible selon l'une quelconque des revendications 1 à 5 dans lequel les moyens de réception d'échantillon ont une forme choisie dans le groupe comprenant un tube, un tube comprenant au moins un moyen d'échantillonnage d'échantillon biologique liquide, une cavité ouverte dans laquelle l'échantillon biologique liquide peut être déposé ou une cavité ouverte comprenant au moins un moyen d'échantillonnage d'échantillon biologique liquide.

7. Dispositif lisible selon l'une quelconque des revendications 1 à 6 dans lequel la plateforme comprend au moins une zone transparente (14) dans laquelle le moyen visuel d'indication de volume est positionné.

8. Dispositif lisible selon l'une quelconque des revendications 1 à 7 comprenant au moins un moyen d'identification lisible dans lequel des informations sont codées, lesdites informations sont choisies dans le groupe comprenant le type du dispositif lisible, la date de fabrication, la date de validité du dispositif lisible ou toute combinaison de ceux-ci.

9. Dispositif de lecture (20) selon la revendication 1 dans lequel le moyen de reconnaissance est raccordé à un moyen de signalisation pour la fourniture à l'utilisateur d'un signal informatif relatif à la présence et/ou à la compatibilité du dispositif lisible avec le dispositif de lecture (20), ledit moyen de signalisation est un moyen visuel et/ou auditif.

10. Dispositif de lecture (20) selon la revendication 9 dans lequel le couvercle (23) comprend au moins une ouverture pouvant se fermer à travers laquelle au moins un moyen de réception d'échantillon du dispositif lisible est accessible.

11. Dispositif de lecture (20) selon l'une quelconque des revendications 9 à 10 comprenant au moins une interface utilisateur (21) qui est raccordée à tout élément du dispositif de lecture (20), ladite interface fournit et/ou reçoit des informations en provenance de l'utilisateur, lesdites informations sont choisies dans le groupe comprenant la sélection d'un programme de vitesse de rotation, le démarrage de la rotation, la présentation des résultats sous forme lisible, l'indication d'un message d'erreur.

12. Procédé de réalisation d'analyses d'immuno-essais comprenant les étapes de :
a) introduction d'au moins un échantillon biologique liquide dans les moyens de réception d'échantillon d'un dispositif lisible selon l'une quelconque des revendications 1 à 8,
b) insertion du dispositif lisible dans le logement (24) d'un dispositif de lecture (20) selon la revendication 1 et selon l'une quelconque des revendications 9 à 11, les étapes (a) et (b) étant interchangeables,
c) écoulement de l'échantillon biologique liquide dans les unités d'analyse par rotation du dispositif lisible par le moyen de rotation du dispositif de lecture (20), et
d) détermination du résultat de l'analyse d'immuno-essai par le premier capteur optique du dispositif de lecture (20), ledit premier capteur optique détecte la présence d'au moins une paire de molécules de liaison dans le moyen de séparation du dispositif lisible et/ou la présence de molécules libres dans la chambre de détection du dispositif lisible,
dans lequel le volume de l'échantillon biologique liquide introduit dans les moyens de réception d'échantillon est détecté par :
- le moyen visuel d'indication de volume du dispositif lisible, et/ou
- le moyen d'indication de volume lisible électroniquement du dispositif lisible et le second capteur optique du dispositif de lecture (20).

13. Procédé selon la revendication 12 dans lequel lorsque l'étape (b) est réalisée avant l'étape (a), l'échantillon biologique liquide est introduit dans les moyens de réception d'échantillon du dispositif lisible à travers l'ouverture pouvant se fermer du couvercle (23) du dispositif de lecture (20).

14. Procédé selon l'une quelconque des revendications 12 ou 13 comprenant en outre l'étape de fourniture d'un message à l'utilisateur, ledit message comprend des informations sur la détection du volume de l'échantillon biologique liquide par le second capteur optique du dispositif de lecture (20).
